# DEMANDE DE BREVET EUROPEEN

(11) **EP 4 101 863 A1**
(43) Date de publication de la demande: **14.12.2022**
(21) Numéro de dépôt: 22182904.7
(22) Date de dépôt: 16.10.2020
(51) Int. Cl.: C07K 14/395, C12N 1/18, C12G 1/00

(54) **LEVURES OENOLOGIQUES POUR LE CONTROLE DE LA TENEUR EN ACIDE MALIQUE DES VINS**

(30) Priorité: 17.10.2019 FR 1911637
(62) Demande divisionnaire de: 20803632.7
(71) Demandeur: Biolaffort, 33270 Floirac (FR)
(72) Inventeur: PELTIER, Emilien, 33290 PAREMPUYRE (FR); MARULLO, Philippe, 33370 TRESSES (FR); COULON, Joana, 33130 BEGLES (FR)
(74) Mandataire: Ipside

(57) **Abrégé**

L'invention concerne l'utilisation, dans la fermentation alcoolique du jus de raisin en vin, d'une souche de levure de l'espèce *Saccharomyces cerevisiae* comprenant au moins le polymorphisme de séquence peptidique Asp200Glu dans la protéine PMA1 codée par le gène YGL008C. Cette souche permet de diminuer le pH du vin fabriqué par le contrôle de sa teneur en acide malique.

## Description

La présente invention est du domaine des levures destinées à être utilisées dans l'industrie agroalimentaire, et vise en particulier des levures destinées à être utilisées dans la biotransformation du jus de raisin en vin. Le jus de raisin adapté à la vinification étant communément qualifié de moût de raisin. La présente invention concerne plus particulièrement, mais non exclusivement, un procédé de sélection de levains du genre *Saccharomyces* et en particulier de l'espèce *Saccharomyces cerevisiae* afin de mieux contrôler le pH du produit de la fermentation du jus de raisin en vin et ceci en sélectionnant leur aptitude à dégrader (ou à produire) des acides organiques et en particulier l'acide malique et l'acide succinique.

La teneur de l'ensemble des acides organiques du vin contribue de manière importante à son acidité finale et donc au pH final ce qui est un paramètre important de sa qualité organoleptique. Le jus de raisin comporte naturellement de nombreuses substances organiques, et entre-autres des acides organiques tels que l'acide malique et l'acide tartrique. L'acidité du jus de raisin et des vins préparés à partir de ce jus, est étroitement dépendante de la teneur de ces deux acides. Les teneurs en acide malique dans le raisin diminuent au cours de sa maturation pour atteindre des valeurs de 2 à 7 g/L au moment de la maturité. De plus, lors de la transformation du jus de raisin en vin, les levures peuvent dégrader partiellement l'acide malique et produire d'autres acides organiques tel que l'acide succinique et l'acide pyruvique qui vont à leur tour modifier l'acidité du vin. A noter que, dans la plupart des vins rouges et certains vins blancs il est important de dégrader complètement l'acide malique afin de maîtriser l'équilibre acide des vins. En oenologie, la teneur en acide malique est principalement contrôlée par la fermentation malo-lactique réalisée par les bactéries lactiques en particulier par *Oenococcus oeni.* Cette fermentation a lieu après la fermentation alcoolique, et peut être initiée en utilisant des levains industriels inoculés dans le vin ou dans le moût en fermentation. Cependant, le succès de l'implantation de telles bactéries dans le vin n'est pas facilement reproductible, surtout dans des conditions extrêmes comme par exemple avec un pH faible, une présence de SO₂, et/ou un degré alcoolique fort.

D'autres méthodes ont été mise en oeuvre, telles que l'expression dans la levure *Saccharomyces cerevisiae* à la fois de l'enzyme malo-lactique de *Oenococcus oeni* et du transporteur d'acide malique de *Schizosaccharomyces pombe,* qui permettent à la levure de dégrader de manière complète l'acide malique lors de la fermentation alcoolique comme cela est décrit dans l'article de Volschenk et al., publié dans Nat. Biotechno/., 1997, Mar, 15(3), 253-257*.* Les contraintes réglementaires liées au statut d'organisme génétiquement modifié (OGM) de la souche de levure, interdisent l'utilisation de cette méthode dans la plupart les pays producteurs de vins, et des alternatives efficaces doivent donc être trouvées. Une de ces alternatives consistant à employer d'autres espèces de levure comme la fermentation du vin par la levure *Schizosaccharomyces pombe* qui est capable de consommer la totalité de l'acide malique, est décrite dans l'article de Taillandier et al., Journal of Siotechnology, 1995, 40, 3, 199-205. Cependant, cette espèce *Schizosaccharomyces pombe* produit des composés indésirables qui ne sont pas compatibles avec la production industrielle de vin, et son utilisation ne peut donc pas être considérée comme une alternative efficace.

Le brevet EP 2 183 364 décrit l'utilisation de l'espèce *Saccharomyces cerevisiae* avec une sélection au sein de la variabilité génétique de l'espèce de caractères oenologiques liés à la production de phénols volatils. L'espèce *Saccharomyces cerevisiae* présente une variabilité génétique naturelle particulièrement attractive, mais jusqu'alors aucun candidat permettant une réduction significative de l'acide malique n'a été convenablement sélectionné et aucune souche pouvant consommer la quasi-totalité de l'acide malique, avec à tout le moins une réduction significative de l'acide malique d'au moins 50% n'a pu être convenablement identifiée. Par ailleurs cette espèce peut dans certain cas produire de faibles quantités d'acide malique tel que décrit dans Yeramian et al. J. Agric. Food Chem., Vol. 55, No. 3, 2007 912-919. Cependant le niveau de production d'acide malique de ces souches reste inférieur ou égal à 0.7 g/L.

Pour remédier à tout ou partie des inconvénients de l'état de la technique précitée, la présente invention concerne une souche de levure de l'espèce *Saccharomyces cerevisiae* comprenant au moins un polymorphisme de séquence peptidique dans au moins une des séquences des protéines codées par les gènes sélectionnés parmi les onze gènes *YLL041C (SDH2), YGL008C (PMA1), YDL054C (MCH1), YKL029C (MAE1), YDL021W (GPM2), YOR380W (RDR1), YOR090C (PTC5), YBR218C (PYC2), YGL037C (PNC1), YBL036C et YDL237W (AIM6),* lesquels sont localisés sur les chromosomes (chr) 2, 4, 7, 11, 12 et 15 . Dans le cadre de l'invention, un polymorphisme d'une séquence peptidique est de préférence lié à un polymorphisme unique de séquence (connu sous l'acronyme anglo saxon « SNP », signifiant « single nucleotide polymorphism ») ou une succession de plusieurs polymorphismes unique de séquence au niveau de la séquence nucléique du gène codant pour cette protéine. Les séquences génétiques et/ou peptidiques sont celles identifiées dans la base de donnée *Saccharomyces* GENOME DATABASE (SGD) pour la souche S288C et correspondent, plus particulièrement, aux séquences détaillées dans la partie « liste de séquences » (*sequence listing*) de la présente description.

Les inventeurs ont mis en évidence de manière tout à fait inattendue que l'utilisation de marqueurs génétiques permettait d'accélérer la sélection d'individus performants sans réaliser des tests fermentaires. Une telle sélection permet d'obtenir pour sensiblement la moitié des individus concernés, une nette modification du taux d'acide malique consommé et une modification du pH du vin résultant.

Avantageusement, la souche de levure selon l'invention comprend au moins un, de préférence de un à six, polymorphismes de séquence peptidique au niveau des acides aminés sélectionnés parmi les positions suivantes dans le génome de référence : l'acide l'acide aminé (Lys158) de la protéine codée par le gène *YLL041C,* l'acide aminé (Pro74) de la protéine codée par le gène *YGL008C,* l'acide aminé (Asp200) de la protéine codée par le gène *YGL008C,* l'acide aminé (Ser63) de la protéine codée par le gène *YDL054C,* l'acide aminé (Ile605) au niveau de la protéine codée par le gène *YKL029C,* l'acide aminé (Met419) au niveau de la protéine codée par le gène *YOR090C,* l'acide aminé (Glu722) de la protéine codée par le gène *YBR218C* et l'acide aminé (Glu165) de la protéine codée par le gène *YDL237W.* De préférence, ladite souche comporte le polymorphisme de séquence peptidique au niveau de l'acide aminé (Asp200) de la protéine codée par le gène *YGL008C.*

Avantageusement, la souche de levure de l'espèce *Saccharomyces cerevisiae* comprend au moins un polymorphisme de séquence peptidique dans la séquence de chacune des protéines codées par les deux gènes *YLL041C* et *YGL00*8*C* localisés sur les chromosomes 7 et 12 Une telle sélection des individus permet d'obtenir une teneur d'acide malique consommée moyenne, qui est prise sur l'ensemble de la population, et qui est supérieure à 51%, en étant exprimée en pourcentage massique calculé par rapport à la masse totale d'acide malique présent au départ. De préférence, ladite souche comprend deux polymorphismes de séquence peptidique au niveau de deux acides aminés sélectionnés parmi les positions suivantes dans le génome de référence : l'acide aminé (Lys158) de la protéine codée par le gène *YLL041C,* l'acide aminé (Pro74) de la protéine codée par le gène *YGL008C,* l'acide aminé (Asp200) de la protéine codée par le gène et *YGL008C* et l'acide aminé (Ile605) au niveau de la protéine codée par le gène *YKL029C.*

Avantageusement, la souche de levure de l'espèce *Saccharomyces cerevisiae* selon l'invention comprend au moins un polymorphisme de séquence peptidique dans la séquence de chacune des protéines codées par les quatre gènes *YLL041C, YGL008C, YDL054C* et *YDL237W* localisés sur les chromosomes 4, 7 et 12 . Une telle sélection des individus permet d'obtenir une teneur d'acide malique moyenne (prise sur l'ensemble de la population) consommée supérieure à 54% calculée par rapport à la masse totale d'acide malique présent au départ. De préférence, ladite souche comprend quatre polymorphismes de séquence peptidique au niveau de quatre acides aminés sélectionnés parmi les positions suivantes dans le génome de référence : l'acide aminé (Lys158) de la protéine codée par le gène *YLL041C,* l'acide aminé (Pro74) de la protéine codée par le gène *YGL008C,* l'acide aminé (Ser63) de la protéine codée par le gène *YDL054C* et l'acide aminé (Glu165) de la protéine codée par le gène *YDL237W.*

Avantageusement, la souche de levure de l'espèce *Saccharomyces cerevisiae* selon l'invention comprend au moins un polymorphisme de séquence peptidique dans chacune des séquences de cinq à six protéines codées par les sept gènes *YLL041C, YGL008C, YDL054C, YKL029C, YOR090C, YBR218C* et *YDL237W* localisés sur les chromosomes 2, 4, 7, 11, 12 et 15. Une telle sélection des individus permet d'obtenir une teneur d'acide malique consommée supérieure à 58.5% (prise sur l'ensemble de la population) calculée par rapport à la masse totale d'acide malique présent au départ.

De préférence, ladite souche comprend cinq polymorphismes de séquence peptidique au niveau de cinq acides aminés sélectionnés parmi les positions suivantes dans le génome de référence : l'acide aminé (Lys158) de la protéine codée par le gène *YLL041C,* l'acide aminé (Pro74) de la protéine codée par le gène *YGL008C,* l'acide aminé (Ser63) de la protéine codée par le gène *YDL054C,* l'acide aminé (Ile605) au niveau de la protéine codée par le gène *YKL029C,* l'acide aminé (Met419) au niveau de la protéine codée par le gène *YOR090C* et l'acide aminé (Glu165) de la protéine codée par le gène *YDL237W.*

De préférence, ladite souche comprend six polymorphismes de séquence peptidique au niveau de six acides aminés sélectionnés parmi les positions suivantes dans le génome de référence : l'acide aminé (Lys158) de la protéine codée par le gène *YLL041C,* l'acide aminé (Pro74) de la protéine codée par le gène *YGL008C,* l'acide aminé (Ser63) de la protéine codée par le gène *YDL054C,* l'acide aminé (Ile605) au niveau de la protéine codée par le gène *YKL029C,* l'acide aminé (Met419) au niveau de la protéine codée par le gène *YOR090C,* l'acide aminé (Glu722) de la protéine codée par le gène *YBR218C* et l'acide aminé (Glu165) de la protéine codée par le gène *YDL237W.*

De préférence, ladite souche comprend de deux à six substitutions d'acides aminés dans le génome de référence parmi les suivantes: (Glu158) de la protéine codée par le gène *YLL041C,* (Leu74) de la protéine codée par le gène *YGL008C,* (Leu63) de la protéine codée par le gène *YDL054C,* (Leu419) de la protéine codée par le gène *YOR090C,* (Lys722) de la protéine codée par le gène *YBR218C* et (Ala165) de la protéine codée par le gène *YDL237W.* Une telle sélection de souche est avantageusement utilisée pour obtenir une forte diminution du taux d'acide malique, et augmenter le pH d'un vin. Une telle sélection des individus permet d'obtenir une teneur d'acide malique consommée au dela de 80% et qui produisent un vin dont le pH est modifié d'une valeur pouvant atteindre 0.4 unités selon les souches à partir du même jus de raisin de départ.

De préférence, ladite souche comprend de une à deux substitutions d'acides aminés dans le génome de référence parmi les suivantes : (Glu200) de la protéine codée par le gène et *YGL008C* et (Val605) au niveau de la protéine codée par le gène *YKL029C.* Une telle sélection de souche permet d'obtenir une forte augmentation d'acide malique, et de diminuer le pH d'un vin. Une telle sélection de souche est avantageusement utilisée pour obtenir une forte augmentation du taux d'acide malique, et diminuer le pH d'un vin. Une telle sélection des individus permet d'obtenir une production d'acide malique supérieure à 35 % de la valeur d'acide malique initialement présent. Cette production permet de diminuer le pH du vin d'une valeur pouvant atteindre 0.4 unités selon les souches utilisées à partir du même jus de raisin de départ.

La présente invention concerne également un procédé de sélection de souches de levures oenologiques telles que décrites précédemment dans le cadre de la présente invention comportant les étapes consistant à :
1- réaliser une hybridation par croisement de deux souches oenologiques A et B de l'espèce *Saccharomyces cerevisiae ;*
2- identifier les allèles responsables de la consommation de l'acide malique en éliminant une des deux copies parentales en réalisant un hybride hémizygote par insertion d'un gène de résistance à un antibiotique ;
3- obtenir des descendants méiotiques issus des hybrides de l'étape 1-par l'induction de la sporulation et isolement des spores ;
4- déterminer le phénotype des descendants obtenus dans l'étape 3-pour la consommation d'acide malique ;
5- extraire l'ADN des descendants obtenus à l'étape 3- ;
6- effectuer un génotypage du génome des descendants permettant de connaitre le génotype d'au moins deux locus ;
7- réaliser une cartographie génétique à partir des données obtenues à l'étape 4- et à l'étape 6- ; et
8- sélectionner au moins un groupe d'individus parmi les individus porteurs d'allèles démaliquants et les individus porteurs d'allèles maliquants.

Dans le cadre de la présente invention, le terme « sélection » signifie l'identification et l'isolement de levures oenologiques, c'est-à-dire des levures intervenant dans le processus de fabrication du vin, et notamment dans le métabolisme des acides et des bases présentes dans les vins.

Le terme « allèle démaliquant » signifie l'allèle qui confère une plus forte consommation de la teneur d'acide malique au cours de la fermentation alcoolique. Alternativement, le terme « allèle maliquant » signifie l'allèle qui confère une plus faible consommation de la teneur d'acide malique au cours de la fermentation alcoolique, voire une production d'acide malique au cours de la fermentation alcoolique.

Le terme « génotyper », signifie déterminer l'hérédité d'une portion d'ADN à un point précis du génome. Dans le cadre de la présente invention, le génotypage correspond à l'identification d'une séquence nucléotidique qui est associée au caractère démaliquant ou au caractère maliquant.

L'étape 1- consiste, de préférence, en l'obtention d'un hybride par croisement de deux souches A et B de l'espèce *Saccharomyces cerevisiae* selon la méthode d'appariement de spores au micromanipulateur décrite dans l'article de Marullo et al. (Marullo et al. 2009 FEMS Yeast Research, 9, 8 :1148-1160). L'hybride obtenu est appelé H1, il est le produit de la conjugaison de spores issues du croisement entre la souche A et la souche B. L'hybride H1 obtenu est hétérozygote en de nombreux points de son génome.

Avantageusement, la technique des hybrides hémizygotes mise en oeuvre à l'étape 2- est la méthode décrite dans l'article de Steinmetz et al., 2002, Nature, 416, 6878 :326-330. Le principe de ladite méthode consiste à éliminer par recombinaison homologue une des deux copies parentales du gène étudié, en insérant au locus dudit gène étudié, un gène de résistance à un antibiotique. Ainsi les hybrides hémizygotes n'expriment qu'une seule des deux copies parentales du gène étudié. S'il existe une différence significative (au seuil de 10%) entre les deux hybrides, l'allèle a un effet sur le phénotype étudié. Les gènes testés dont l'effet est significatif sur le pourcentage de consommation d'acide malique en fin de fermentation alcoolique sont présentés dans le tableau 1. L'acide L-malique est quantifié par dosage enzymatique après avoir observé l'arrêt complet de la fermentation alcoolique selon la méthode décrite dans Peltier, et al., 2018, PLOS ONE, 13(1), 191353 (https://doi.org/10.1101/191353). L'allèle noté DEMAL est celui qui consomme le plus d'acide malique, l'allèle noté MAL est celui qui préserve le plus la teneur en acide malique. Les résultats présentés ont été mesurés dans un moût de Merlot (échantillon Merlot 2 du tableau 1) et dans un moût de Sauvignon (échantillon Sauvignon Blanc 1 du tableau 1) dont les teneurs en acides maliques initiales sont respectivement estimées à 2,28 et 4,32 g/L.

**Tableau 1 - la colonne (a) représente le nombre de répétitions par groupes**

| gènes | Mout | Allèle | | % MALIQUE consommé | | DEMAL-MAL | Test WMW | (a)^{∗} |
|---|---|---|---|---|---|---|---|---|
| | | Mal | Demal | Moy allele DEMAL | Moy allele MAL | | | |
| YLL041C | Merlot2 | A | B | 28,9 | 16,7 | 12,2 | *** | 5 |
| YDL021W | Merlot2 | A | B | 34,2 | 25 | 9,2 | * | 14 |
| *YGL008C* | Merlot2 | B | A | 39 | 30,7 | 8,3 | *** | 13 |
| YBR218C | Merlot2 | A | B | 39,5 | 32 | 7,5 | *** | 16 |
| YKL029C | Merlot2 | A | B | 29,4 | 22,8 | 6,6 | * | 19 |
| YOR090C | Merlot2 | A | B | 37,3 | 31,6 | 5,7 | * | 5 |
| YGL037C | Merlot2 | A | B | 52,9 | 43,4 | 9,5 | * | 14 |
| *YGL008C* | Sauvignon 1 | B | A | 36,8 | 32,9 | 3,9 | *** | 6 |
| YBR218C | Sauvignon1 | A | B | 30,3 | 27,8 | 2,5 | * | 7 |

Ainsi les inventeurs ont mis en évidence de manière tout à fait inattendue que l'utilisation de variations génétiques naturelles peut permettre de diminuer ou d'augmenter la teneur en acide malique des vins en fin de fermentation alcoolique.

Avantageusement, l'étape 3- est réalisée par une microdissection ou une purification en masse de spores. L'étape 3- comporte une induction de la sporulation d'un hybride, le susdit hybride H1, sur un milieu comprenant de préférence de l'acétate de potassium à 1% pendant 3 jours à 24° C. L'utilisation de la microdissection des ascospores est de préférence réalisée à l'aide d'un micromanipulateur, par exemple selon la méthode publiée dans Marullo et al., 2009, FEMS Yeast Research, 9, 8 :1148-1160. Alternativement, les ascospores sont isolés en masse après une purification partielle en utilisant de préférence la méthode publiée dans l'article de Curran B.P.G. et al., 2006, « Basic Investigations in Saccharomyces cerevisiae », Methods in Molecular Biology, vol 313, 1-13.

Chacunes des variations hétérozygotes retrouvées chez l'hybride H1 ségrége au sein de sa descendance.

De préférence, à l'étape 4- le niveau d'acide malique consommé est mesuré en fin de fermentation alcoolique par un test enzymatique. La mesure du pourcentage d'acide malique consommé pour un ensemble de 94 descendants de l'hybride H1 est réalisé par dosage enzymatique de l'acide L-malique après avoir observé l'arrêt complet de la fermentation alcoolique selon la méthode décrite dans Peltier et al., 2018, PLOS ONE, 13(1), 191353 (https://doi.org/10.1101/191353).

A l'étape 5- l'ADN des descendants est extrait. L'ADN de chaque descendant est avantageusement extrait en utilisant des kits d'extraction tels que le kit DNA-Wizard Promega selon le protocole décrit dans Marti-Raga et al. 2017, G3 February, 399-412.

Avantageusement, une amplification de l'ADN génomique est réalisée entre les étapes 5- et 6-. Une amplification est réalisée avec un kit, de préférence le kit Nextera XT ou tout autre kit adapté pour le séquençage de petits génomes. La procédure utilisée permet d'indexer grâce à des amorces d'ADN spécifiques, 96 individus et d'amplifier leur ADN afin de construire une Library pour un séquençage Illumina. Le protocole utilisé est celui fourni par le fabricant, il est décrit dans Marti-Raga et al. 2017, G3 February, 399-412.

Avantageusement, le génotypage effectué à l'étape 6- met en oeuvre un séquençage, lequel séquençage est réalisé de préférence à l'aide d'un kit de séquençage qui consiste dans un premier temps à amplifier l'ADN afin de construire une Library pour le séquençage Illumina MySeq à l'aide du kit de séquençage (Pair End 2x250 bases) MySeq Reagent Kit V2 selon les prescriptions fournies par le constructeur comme décrit dans Marti-Raga et al. 2017, G3 February, 399-412. Les lectures de séquences obtenues sont de préférence filtrées par des méthodes bioinformatiques en utilisant la suite FASTX-Toolkit (http:// hannonlab.cshl.edu/fastx_toolkit/). Les lectures retenues (qualité Q>20) sont ensuite alignées avec le logiciel BWA (Li, H., and R. Durbin, 2010. Bioinformatics 26(5): 589-595) sur le génome de référence S288C (sacCer3, version avril 2011). A l'aide de la suite d'outils SAM tools décrite dans l'article de Li, H. et al., 2009, Bioinformatics, 25, 2078-2079, les SNP de chacun des descendants sont identifiés et positionnés sur le génome par le logiciel Pileup. Ces SNP permettent la construction d'une carte génétique très dense constituée de 1000 marqueurs bi alléliques. La méthode de construction de ce type de carte et les outils bioinformatiques utilisés sont décrits dans l'article Marti-Raga et al. 2017, G3 February, 399-412.

Avantageusement, la cartographie génétique utilisée à l'étape 7- du procédé selon l'invention est une méthode qui établit un lien statistique entre l'hérédité de marqueurs génétiques et la variation d'un caractère quantitatif. Les marqueurs génétiques qui sont statistiquement liés sont des « locus de caractère quantitatifs » (dont la traduction anglaise est « Quantitative Trait Loci », avec l'acronyme associé « QTL ») tels que ceux décrit dans la demande EP 2 183 364. Les méthodes de détection de QTL chez les levures sont décrites dans la littérature, par exemple dans l'article publié par Peltier et al., 2018, BMC Genomics 19, 772. A l'étape 7- les marqueurs bi-alléliques (ils sont de l'ordre du millier) de l'étape 6- sont utilisés pour réaliser la cartographie génétique. Un lien statistique est trouvé entre la variation du caractère quantitatif et quatre marqueurs génétiques avec un seuil de significativité très important (II_150, XV_1052, IV_31 et IV_360). Un seuil de significativité avec un taux de fausse découverte de 5% est calculé à partir des données permutées : il est de pval = 0.0004 pour le pourcentage d'acide malique consommé. Le test statistique utilisé est basé sur cartographie par la méthode dite de « *interval mapping*» en utilisant une régression de Haley-Knott (Peltier et al. 2018., BMC Genomics 19, 772). La position génomique des marqueurs, le gène concerné et la valeur de la probabilité du test (risque de première espèce) sont listés dans le tableau 2. Pour chaque marqueur on indique également l'effet de l'allèle MAL et DEMAL exprimé en pourcentage d'acide malique consommé. L'allèle DEMAL correspondant au « caractère démarquant » qui signifie l'allèle qui confère le phénotype responsable de l'élimination de la plus forte teneur d'acide malique (à l'inverse de l'allèle MAL).

**Tableau 2 - ^{∗}NP : non pertinent**

| nom du MARQUEUR | II_150 | XV_1052 | IV_31 | IV_360 |
|---|---|---|---|---|
| P value (-log10) avec un seuil de fausse découverte de 0.05 | 8.89 | 4.28 | 4.35 | 6.98 |
| Gene impacté | YBL036C | YOR380W | YDL237W | YDL054C |
| chromosome | chr2 | chr15 | chr4 | chr4 |
| position | 150422 | 1052746 | 31151 | 360451 |
| référence (S288C) | A | G | A | G |
| allèle MAL | T(Souche A) | A (Souche B) | A (Souche A) | A (Souche B) |
| allèle DEMAL | A (Souche B) | G (Souche A) | C (Souche B) | G (Souche A) |
| Acide aminé modifié allèle MAL | NP^{∗} | H486 | E165 | S279 |
| Acide aminé modifié allèle DEMAL | NP^{∗} | R486 | A165 | P279 |
| % consommé allèle MAL en Sauvignon Blanc 1 | 26.3 | 26.6 | 26.8 | 25.6 |
| % consommé allèle DEMAL en Sauvignon Blanc 1 | 33.3 | 31 | 33,1 | 33.5 |
| % consommé allèle MAL en Merlot 2 | 25,8 | 27 | 27,6 | 24.3 |
| % consommé allèle DEMAL en Merlot 2 | 32,3 | 30.4 | 33,3 | 33.8 |
| DEMAL-MAL (Sauvignon Blanc 1) (%) | 7 | 34.4 | 6,3 | 7,9 |
| DEMAL-MAL (Merlot 2) (%) | 6,5 | 3,4 | 5,7 | 9,5 |

Ainsi, à partir des résultats obtenus suite à l'identification des allèles MAL et DEMAL (étape 2- du procédé) et de la cartographie des QTL chez l'hybride H1 (étapes 3- à 7-) les gènes et/ou les QTL présentant des variations nucléotidiques qui sont liées au pourcentage massique de consommation de l'acide malique au cours de la fermentation alcoolique du jus de raisin par la levure *Saccharomyces cerevisiaes* ont directement identifiés.

Ces variations nucléotidiques affectent la séquence des gènes *YDL237W, YDL054C, YKL029C, YDLO21W, YOR380W, YBR218C, YOR090C,YLL041C, YGL037C, YGL008C* et *YEL036C.* Ces variations de séquence nucléotidique peuvent être aisément génotypées au sein d'une large population d'individus par des méthodes de typage génétique, c'est-à-dire toute méthode de biochimie ou de biologie moléculaire capable de renseigner la présence d'une variation de séquence nucléotidique ou protéique tel que la PCR, le séquençage d'ADN, le séquençage de protéine, et/ou une méthode basée sur la détection de SNP mettant en oeuvre de la spectrométrie de masse, selon la technologie qualifiée de *sequenom^{®}* Gabriel, S., 2009, Current Protocols in Human Genetics, Suppl. (60. doi.org/10.1002/ 0471142905.hg0212s60).

A partir de la séquence génomique des souches A et B de l'espèce *Saccharomyces cerevisiae,* présentées précédemment dans le cadre de l'invention, certaines variations nucléotidiques qui affectent la séquence des protéines codées par les gènes présentés précédemment dans le cadre de l'invention (*YDL237W, YDL054C, YKL029C, YDL021W, YOR380W, YBR218C, YOR090C, YLL041C, YGL037C, YGL008C* et *YBL036C*)*,* sont déduites. Le tableau 3 établit le lien entre les allèles et un phénotype observé.

Les allèles MAL et DEMAL utilisés sont déterminés à partir de la séquence de référence sacCer3 (sacCer3, version avril 2011) issue de la souche S288C et disponible sur la base de données SGD (*« Saccharomyces Genome Database*») avec un lien accessible via https://www.yeastgenome.org/. La nomenclature utilisée est celle de la description des mutations génétiques décrite dans l'article de den Dunnen JT et al., 2000, *Human Genome Variation Society,* Hum.Mutat. 15:7-12.

**Tableau 3**

| Locus | GENE | Nom systématique | Allele DEMAL | Allele MAL |
|---|---|---|---|---|
| II_661 | *PYC2* | *YBR218C* | p.Glu722Lys | p.Glu722 |
| IV_360 | *MCH1* | *YDL054C* | p.Ser63Leu | p.Ser63 |
| VII_480 | *PMA1* | *YGL008C* | p.Pro74Leu | p.Asp200Glu |
| XI_382 | *MAE1* | *YKL029C* | p.Ile605 | p.Ile605Val |
| XII_53 | *SDH2* | *YLL041C* | p.Lys158Glu | p.Lys158 |
| XV_491 | *PTC5* | *YOR090C* | p.Met419Leu | p.Met419 |
| IV_31 | *AIM6* | *YDL237W* | p.Glu165Ala | p.Glu165 |
| VI_427 | *PNC1* | *YGL037C* | p.Val112Ala | p.Val112 |
| IV_414 | *GPM2* | *YDL021W* | p.Thr2IIIe | p.Thr2 |
| II_150 | - | *YBL036C* | g.150422A | g.150422T |
| XV_1052 | *RDR1* | *YOR380W* | p.Gly79 | p.Gly79Asp |

Afin d'utiliser ces variations de séquences nucléotidiques pour améliorer les souches de levure, on détermine des pools d'allèles qui vont permettre de diminuer ou de préserver la teneur en acide malique des vins en fin de fermentation alcoolique. Plusieurs pools d'allèles sont définis et sont désignés par les acronymes suivants : Q2demal, Q2mal, Q4demal, Q4mal, Q6demal, Q6mal, Q7demal, Q7mal, Q6c et Q7c. Les chiffres (2, 4, 6 ou 7) correspondent au nombre de locus pris en compte dans chaque pool. Les pools Q2demal, Q4demal, Q6demal et Q7demal contiennent uniquement des allèles liés au caractère démaliquant. Les pools Q2mal, Q4mal, Q6mal et Q7mal contiennent uniquement des allèles liés au caractère maliquant. Les pools Q6c et Q7c contiennent le pools d'allèles Q4demal complétés avec respectivement 2 et 3 allèles maliquants.

### Pools de deux allèles

Q2demal: *YLL041C* (p.Lys158Glu) + *YGL008C* (p.Pro74Leu)
Q2mal: *YLL041C* (p.Lys158) + *YGL008C* (p.Asp200Glu)

### Pools de 4 allèles

Q4demal : *YLL041C* (p.Lys158Glu) + *YGL008C* (p.Pro74Leu) + *YDL054C* (p.Ser63Leu) + *YDL231W* (p.Glu165Ala)
Q4mal : *YLL041C* (p.Lys158) + *YGL008C* (p.Asp200Glu) + *YDL054C* (p.Ser63) + *YDL231W* (p.Glu165)

### Pools de 6 allèles

Q6demal : *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605) + *YOR090C* (p.Met419Leu)
Q6c: *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605Val) + *YOR090C* (p.Met419)
Q6mal : *YLL041C* (p.Lys158) + *YGL008C* (p.Asp200Glu) + *YDL054C* (p.Ser63) + *YDL237W* (p*.*Glu165) + *YKL029C* (p.Ile605Val) + *YOR090C* (p.Met419)

### Pool de 7 allèles

Q7demal : *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605) + *YOR090C* (p.Met419Leu) + *YBR218C* (p.Glu722Lys)
Q7c: *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605Val) + *YOR090C* (p.Met419) + *YBR218C* (p.Glu722)
Q7mal : *YLL041C* (p.Lys158) + *YGL008C* (p.Asp200Glu) + *YDL054C* (p.Ser63) + *YDL237W* (p.Glu165) + *YKL029C* (p.Ile605Val) + *YOR090C* (p.Met419) + *YBR218C* (p.Glu722)

A l'étape 8- le procédé selon l'invention comporte avantageusement un génotypage mettant en oeuvre une détection de polymorphisme au niveau d'un nucléotide (SNP) par spectrométrie de masse, de préférence selon la technologie qualifiée de *sequenom*^{®} (sequenom^{®}iPLEX^{®} Gold) avec l'appareillage MassARRAY^{®} Analyzer Chip Prep Module 384, commercialisé par la société Agena Biosciences^{®} et selon le protocole décrit dans l'article de Gabriel S. et al., 2009, Current Protocols in Human Genetics, Suppl. 60. (doi.org/10.1002/ 0471142905.hg0212s60). Le design des primers est réalisé par l'outil MassARRAY^{®} Assay Design (version 4.0.0.2) permettant d'amplifier des fragments de moins de 120 bases et en sélectionnant des différences de masse entre allèles comprises entre 16 et 70 Da. Environ 15 ng d'ADN sont nécessaires pour génotyper les individus en utilisant la plateforme MassARRAY^{®} iPLEX^{®} (Agena Biosciences^{®}).

Le procédé selon l'invention comporte avantageusement une étape d'isolation d'au moins une souche.

Le procédé selon l'invention comporte avantageusement une étape d'isolation d'au moins deux souches, respectivement désignées par les lettres X et Z, par exemple les clones X28 et Z8, issus de la descendance de deux populations issues d'hybrides obtenus : par exemple, en croisant les souches A et B d'une part, et des souches C et D d'autre part. Les hybrides et leur descendance sont obtenus selon les étapes 1 et 3 telles que décrites précédemment dans le cadre de l'invention.

Avantageusement, lesdits deux clones X28 et Z8, obtenus selon le procédé selon l'invention, sont croisés et l'hybride résultant est mis à sporuler pour obtenir une vaste descendance en mettant en oeuvre les étapes 1 et 3 telles que décrites précédemment. L'ADN de cette descendance est extrait par une méthode décrite par Albertin et al. qui permet une extraction rapide compatible avec la technique de génotypage du *sequenom^{®}* (Albertin et al., 2018, Yeast, 35, 141-156). La descendance et ensuite génotypée par la technologie qualifiée de *sequenom^{®}* décrite en détail dans la partie expérimentale. La sélection de clones ayant un génotype approprié permet de choisir des individus au phénotype extrême sans avoir recours à des tests fermentaires.

La présente invention concerne aussi une utilisation d'une levure de *Saccharomyces cerevisiae* comportant une souche telle que décrite précédemment dans le cadre de la présente invention pour modifier le pH d'un vin, et de préférence pour augmenter le pH d'un vin.

De manière alternative à ce qui précède, le procédé selon l'invention comporte avantageusement une étape d'isolation d'au moins deux souches, respectivement désignées par les lettres X et Z, par exemple les clones X8 et Z1, issus de la descendance de deux populations issues d'hybrides obtenus : par exemple, en croisant les souches A et B d'une part, et des souches C et D d'autre part. Les hybrides et leur descendance sont obtenus selon les étapes 1 et 3 telles que décrites précédemment dans le cadre de l'invention.

Avantageusement, lesdits deux clones X8 et Z1, obtenus selon le procédé selon l'invention, sont croisés et l'hybride résultant est mis à sporuler pour obtenir une vaste descendance en mettant en oeuvre les étapes 1 et 3 telles que décrites précédemment. L'ADN de cette descendance est extrait par une méthode décrite par Albertin et al. qui permet une extraction rapide compatible avec la technique de génotypage du *sequenom^{®}* (Albertin et al., 2018, Yeast, 35, 141-156). La descendance et ensuite génotypée par la technologie qualifiée de *sequenom^{®}* décrite en détail dans la partie expérimentale. La sélection de clones ayant un génotype approprié permet de choisir des individus au phénotype extrême sans avoir recours à des tests fermentaires.

La présente invention concerne aussi une utilisation d'une levure de *Saccharomyces cerevisiae* comportant une souche telle que décrite précédemment dans le cadre de la présente invention pour diminuer le pH d'un vin.

La partie expérimentale qui suit détaille les protocoles mis en oeuvre.

### PARTIE EXPERIMENTALE

### Séquences

Les séquences dénommées :
*YDL237W,*
*YDL054C,*
*YKL029C,*
*YDL021W,*
*YOR380W,*
*YBR218C,*
*YOR090C,*
*YLL041C,*
*YGL008C,*
*YGL037C,* et
*YBL036C,*
sont celles décrites dans la base de donnée SGD (Saccharomyce Genome Database - www.yeastgenome.org-).

### Matériels et Méthodes

**Obtention d'un hybride H1** : par croisement de deux souches A et B de l'espèce *Saccharomyces cerevisiae* selon une méthode d'appariement de spores décrite dans l'article de Marullo, P. et al., 2009, FEMS Yeast Research, 9, 8 :1148-1160: l'hybride H1 obtenu est le produit de la conjugaison de spores issues du croisement entre la souche A et la souche B.

**Obtention d'un hybride H2** : par croisement de deux souches C et D de l'espèce *Saccharomyces cerevisiae* selon une méthode décrite dans Marullo, P. et al., 2009, FEMS Yeast Research, 9, 8 :1148-1160: l'hybride H2 obtenu est le produit de la conjugaison de spores issues du croisement entre la souche C et la souche D.

**Isolation d'un clone X28** : A partir de l'hybride H1 issu du croisement des souches A et B on isole le descendant X28 issu d'une population de 94 descendants isolés par micromanipulation (Marullo et al., 2009, FEMS Yeast Research, 9, 8 :1148-1160). Cette technique consiste à disséquer des asques de levures formés à partir d'une culture sur milieu Acétate de Potassium ACK pendant 3 jours à 24° C. Les asques sont digérés par de la cytohélicase (2mg/mL) et les spores sont isolées à l'aide d'un micromanipulateur (Singer^{™} MS200). La teneur en acide malique des 94 descendants est mesurée en fin de fermentation alcoolique, laquelle fermentation est réalisée de la manière suivante :

Les fermentations ont été réalisées dans des flacons de 20 mL commercialisés par la société Fisher Scientific^{®} sous la référence 20-HSV. Ils ont été utilisés pour fermenter 10 mL de moût de l'échantillon Merlot 2 dont la teneur en acide malique de départ est de 2,280 g/L. Les flacons ont été fermés par des bouchons à vis avec un septum en silicone / PTFE HT de 3 mm commercialisé par la société Fisher Scientific^{®}. Des aiguilles hypodermiques ont été insérées dans le septum pour le dégagement du CO₂. Les fermentations ont été initiées avec l'inoculation de 2.10E6 cellules viables.mL⁻¹ de culture en phase liquide (YPD) réalisée dans des microplaques deepwell de 1 mL commercialisées par la société Fisher Scientific^{®}. La concentration de cellules viables a été estimée par cytométrie en flux en utilisant un appareil de la marque Cell Lab Quanta^{™} commercialisé par la société Beckman Coulter^{®}. La température de fermentation a été maintenue à 24° C à l'aide d'un incubateur vendu par la société Binder^{™} GmbH. Les flacons sont placés sous agitation à 175 tr. / min. au cours de la fermentation à l'aide d'un agitateur orbital tel que celui commercialisé par la société Stuart^{®} sous la référence SSL1. Le détail de la méthode est donnée dans l'article de Peltier et al. , 2018, PLOS ONE, 13(1), 191353. (https://doi.org/10.1101/191353).

Les pourcentages de consommation indiqués dans la présente description sont calculés par rapport à la teneur totale d'acide malique présent au départ dans l'échantillon considéré.

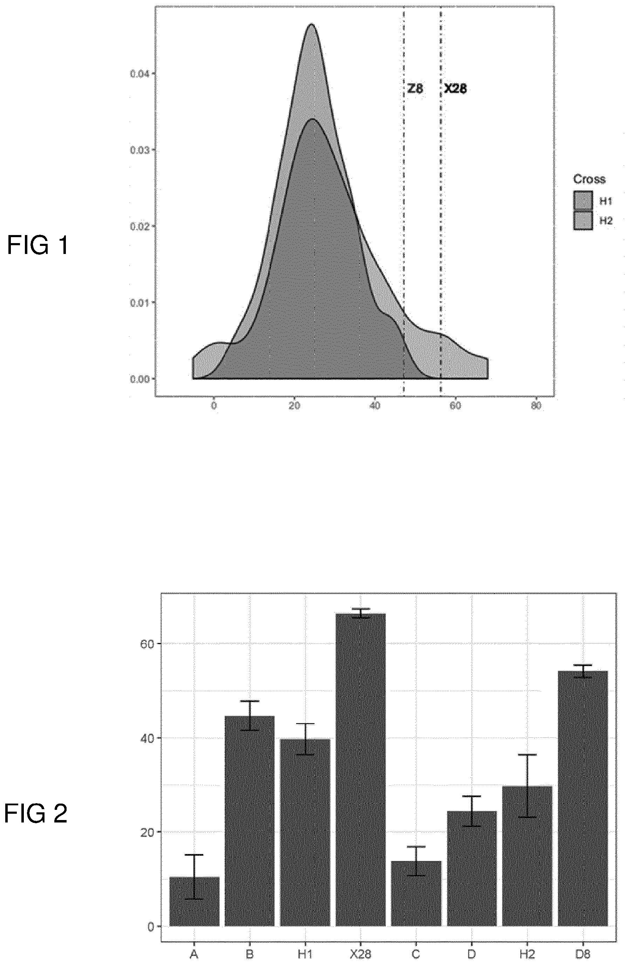

La distribution de la teneur en acide malique consommé - en abscisse, exprimée en pourcentage massique calculé par rapport à la masse totale d'acide malique présent au départ - est montrée à la figure 1 pour la descendance de H1 et en particulier pour le clone X28 dans le moût de Merlot de l'échantillon Merlot 2.

**Isolation d'un clone Z8** : De la même manière que pour l'hybride H1 on obtient l'hybride H2 issu du croisement de la souche C et D. De sa descendance (obtenue comme décrit ci-dessus dans le cadre de l'obtention de X28) on isole le clone Z8 qui présente également un phénotype extrême pour la consommation d'acide malique. La distribution de la teneur (ladite teneur étant exprimée en pourcentage massique calculé par rapport à la masse totale d'acide malique présent au départ) en acide malique consommé est montrée sur la figure 1 pour la descendance de H2 et en particulier pour le clone Z8 dans le moût de Merlot de l'échantillon merlot 2.

La souche B est une souche qui dégrade le plus d'acide malique avec 45% d'acide malique dégradé en comparaison avec les souches A, C et D. Le niveau de consommation d'acide malique des souches X28 et Z8 est reporté à la figure 2 (dans laquelle les valeurs lues en ordonnée correspondent au pourcentage en acide malique consommé moyen dans un moût de Merlot (Merlot 2) et de Sauvignon Blanc (Sauvignon Blanc 1) dont les teneurs en acide malique initiales sont respectivement estimées à 2,28 et 4,32 g/L) ; les valeurs de consommation d'acide malique sont également indiquées pour les quatre souches parentales A, B, C et D et pour les hybrides H1 et H2. La figure 2 montre que le phénotype de consommation est extrême pour les souches X28 et Z8, en comparaison avec le phénotype de consommation des autres souches. Ainsi, le clone X28 sélectionné montre un pourcentage de consommation de 66% ce qui illustre l'efficacité des méthodes de croisement/sélection mises en oeuvre dans le cadre de l'invention pour améliorer les caractères quantitatifs.

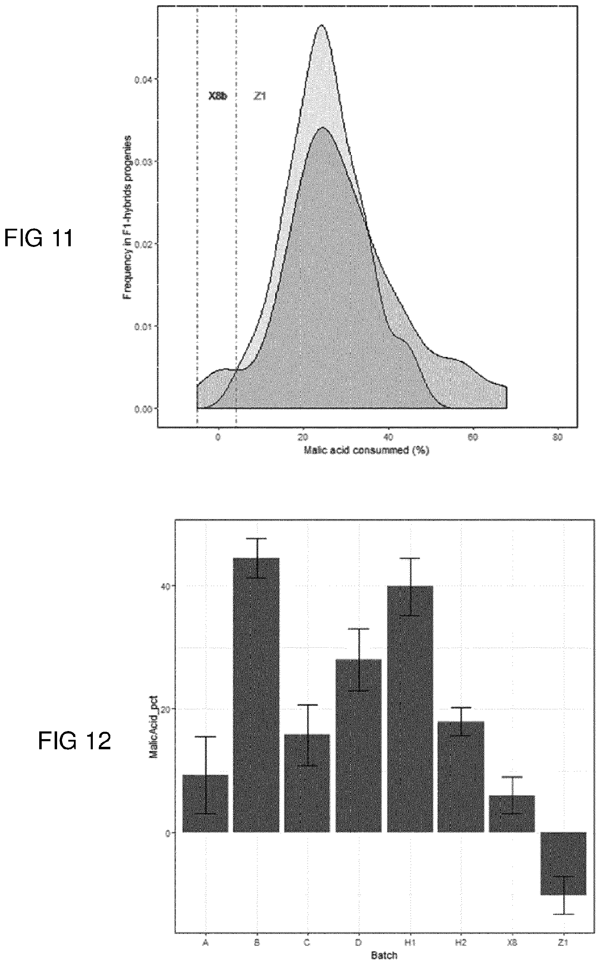

**Isolation d'un clone X8** : A partir de l'hybride H1 issu du croisement des souches A et B on isole le descendant X8 issu d'une population de 94 descendants isolés par micromanipulation (Marullo et al., 2009, FEMS Yeast Research, 9, 8 :1148-1160) de la même façon que celle utilisée pour l'obtention du descendant X28. La teneur en acide malique final exprimée en pourcentage massique calculé par rapport à la masse totale d'acide malique présent au départ - est montrée à la figure 11 pour la descendance de H1 et en particulier pour le clone X8 dans le moût de Merlot de l'échantillon Merlot 2. Le descendant X8 présente une très faible consommation d'acide malique par rapport à la teneur initiale.

**Isolation d'un clone Z1** : De la même manière que pour le descendant Z8 de 1"hybride H2 on isole le clone Z1 qui présente également un phénotype extrême. La distribution de la teneur en acide malique finale (ladite teneur étant exprimée en pourcentage massique calculé par rapport à la masse totale d'acide malique présent au départ) est montrée sur la figure 12 pour la descendance de H2 et en particulier pour le clone Z1 dans le moût de Merlot de l'échantillon merlot 2. Le descendant Z8 produit une faible quantité d'acide malique en fin de fermentation ce qui est un caractère remarquable.

Le niveau de consommation d'acide malique des souches X8 et Z1 est reporté à la figure 12 (dans laquelle les valeurs lues en ordonnée correspondent au pourcentage en acide malique consommé moyen dans un moût de Merlot (Merlot 2) et de Sauvignon Blanc (Sauvignon Blanc 1) dont les teneurs en acide malique initiales sont respectivement estimées à 2,28 et 4,32 g/L) ; les valeurs de consommation d'acide malique sont également indiquées pour les quatre souches parentales A, B, C et D et pour les hybrides H1 et H2. La figure 12 montre que le phénotype de consommation est extrême pour les souches X8 et Z1, en comparaison avec le phénotype de consommation des autres souches. Ainsi, le clone X8 sélectionné montre une légère production d'acide malique en fin de fermentation ce qui se traduit par une teneur negative du pourcentage de consommation de -10%.

**Mise en œuvre de la technique des hybrides hemizygotes.** La technique des hybrides hémizygotes est ensuite mise en oeuvre selon la méthode décrite dans l'article de Steinmetz et al., 2002, Nature, 416, 6878 :326-330. Une recombinaison homologue permet d'éliminer une des deux copies parentales du gène étudié en insérant une cassette conférant à la levure la résistance à la généticine (G418) : à partir de l'hybride H1, les allèles testés sont remplacés par des cassettes de délétion Kan-MX4 qui confèrent une résistance à la généticine (G418), comme cela est décrit dans l'article de Goldstein et al., 1999, - Yeast-Wiley Online Library., 15(14), 1541-1553. Ces cassettes sont amplifiées par PCR (acronyme anglo-saxon de « Polymerase Chain Reaction ») en utilisant comme matrice l'ADN génomique des souches de la collection de délétion Euroscarf (http://euroscarf.de) préalablement délétées pour le gène en question. Les cassettes ont été amplifiées avec les 500 bases en amont et en aval du gène visé, en 5' et 3' de la séquence codante. L'hybride H1 a été transformé avec les cassettes en utilisant le protocole décrit par Gietz, et al., 2007, Nature Protocols, 2(1), 31-34. (https://doi.org/10.1038/nprot.2007.13). L'insertion correcte des cassettes dans les mutants obtenus a été vérifiée par l'amplification positive par PCR d'un fragment qui comporte une centaine de paires de bases (pb) en amont de la zone d'insertion de la cassette, et se terminant au milieu de la cassette. Avec cette stratégie, une transformation réussie remplace l'un des deux allèles de l'hybride. Pour les transformants obtenus, l'identité de l'allèle restant a été déterminée par : - la méthode de RFLP (de l'acronyme anglo-saxon de « *Restriction Fragment Length Polymorphism* ») telle que décrite dans l'article de Botstein D. et al., 1980, Am. J. Hum. Genet., 32:314-331 ; ou - la méthode de Sanger telle que décrite dans l'article de Sanger, F. et al. « Nucleotide sequence of bacteriophage phi X174 DNA », Nature, vol. 265, 1977, p. 687-695.

Lorsqu'il existe une différence d'au moins 5% entre les deux hybrides sur le pourcentage d'acide malique consommé, l'allèle est considéré comme ayant un effet sur le phénotype étudié. Les gènes testés dont l'effet est significatif sur le pourcentage de consommation d'acide malique en fin de fermentation alcoolique sont présentés dans le tableau 1. L'allèle noté DEMAL est celui qui consomme le plus d'acide malique, l'allèle noté MAL est celui qui préserve le plus la teneur en acide malique. Les résultats présentés ont été mesurés dans le moût de Merlot 2 et de Sauvignon Blanc 1 dont les teneurs en acides maliques initiales sont respectivement estimées à 2,28 et 4,32 g/L.

### Cartographie de QTL.

L'ADN de chaque descendant est avantageusement extrait en utilisant des kits d'extraction tels que le kit DNA-Wizard Promega selon le protocole décrit dans Marti-Raga et al. 2017, G3 February, 399-412.

Une amplification est réalisée avec un kit, de préférence le kit Nextera XT ou tout autre kit adapté pour le séquençage de petits génomes. La procédure utilisée permet d'indexer grâce à des amorces d'ADN spécifiques 96 individus et d'amplifier l'ADN afin de construire une Library pour un séquençage Illumina. Le protocole utilisé est celui fourni par le fabricant, il est décrit dans Marti-Raga et al. 2017, G3 February, 399-412.

Un génotypage est ensuite effectué qui met en oeuvre un séquençage, lequel séquençage est réalisé à l'aide d'un kit de séquençage qui consiste dans un premier temps à amplifier l'ADN afin de construire une Library pour le séquençage Illumina comme décrit ci-avant. L'amplification décrite précédemment est mise en oeuvre, et, elle est suivie par le séquençage complet du génome de chaque descendant en utilisant un appareillage Illumina MySeq à l'aide du kit de séquençage (Pair End 2x250 bases) MySeq Reagent Kit V2 selon les prescriptions fournies par le constructeur comme décrit dans Marti-Raga et al. 2017, G3 February, 399-412. Les lectures de séquences obtenues sont de préférence filtrées par des méthodes bio-informatiques en utilisant la suite FASTX-Toolkit (http:// hannonlab.cshl.edu/fastx_toolkit/). Les lectures retenues présentent le paramètre de qualité Q>20, ce qui définit un taux d'erreur inférieur à 1%. Ces lectures sont ensuite alignées avec le logiciel BWA (Li, H., and R. Durbin, 2010. Bioinformatics 26(5): 589-595) sur le génome de référence S288C (sacCer3, version avril 2011) disponible sur la base de données SGD (https://www.yeastgenome.org/). A l'aide de la suite d'outils SAM tools décrite dans l'article de Li, H. et al., 2009, Bioinformatics, 25, 2078-2079, les SNP de chacun des descendants sont identifiés et positionnés sur le génome par le logiciel pileup. Ces SNP permettent la construction d'une carte génétique très dense constituée de 1000 marqueurs bi alléliques. La méthode de construction de ce type de carte et les outils bioinformatiques utilisés sont décrits dans l'article Marti-Raga et al. 2017, G3 February, 399-412.

Une cartographie génétique est ensuite réalisée, laquelle cartographie établit un lien statistique entre l'hérédité de marqueurs génétiques et la variation d'un caractère quantitatif. Les marqueurs génétiques qui sont statistiquement liés sont des « locus de caractère quantitatifs » dont la traduction anglaise est « Quantitative Trait Loci », avec un acronyme anglo-saxon communément utilisé qui est « QTL » ; de tels QTL sont notamment décrits dans la demande EP 2 183 364. Les méthodes de détection de QTL chez les levures sont décrites dans la littérature, par exemple dans l'article publié par Peltier et al., 2018, BMC Genomics 19, 772.

Des marqueurs bi-alléliques identifiés lors du génotypage sont utilisés pour réaliser la cartographie génétique. Un lien statistique est trouvé entre la variation du caractère quantitatif et quatre marqueurs génétiques avec un seuil de significativité très important (II_150, XV_1052, IV_31 et IV_360, voir le tableau 2).

**Sélection génétique de la descendance de l'hybride H3:** un croisement entre les clones Z8 et X28 est ensuite effectué pour donner l'hybride H3 selon le même protocole que celui décrit ci-avant pour l'obtention des hybrides H1 et H2. A partir de cet hybride H3, des spores en masse sont isolés par un procédé faisant appel à l'hydrophobicité de leur paroi, et qui est détaillé dans la description qui suit directement. Une préparation de spores est obtenue après 3 jours de culture sur milieu acétate de potassium (ACK) à 1% en masse à une température de 24° C ; puis ces spores sont digérées dans une préparation de cytohélicase (2mg/mL) et les spores sont purifiées en réalisant des lavages successifs à l'eau et avec une solution aqueuse de Nonidet P40 (également connu sous son nom donné par la classification IUPAC: « octylphenoxypolyethoxyethanol ») à 0.01%. Le degré d'enrichissement en spores varie de 80 à 99% selon les échantillons. A partir de cette préparation, on isole des colonies sur boite de pétri. Dans le cas présent, sensiblement 1000 descendants sont isolés. L'ADN de ces descendants est extrait par une méthode décrite par Albertin et al. (Albertin et al., 2018, Yeast, 35, 141-156) qui permet une extraction rapide compatible avec la technique de génotypage du *sequenom^{®}.*

Les phénotypes des 1000 descendants isolés de l'hybride H3 est évalué par la méthode du sequenom^{®} selon le protocole décrit par Gabriel S. et al., 2009, Current Protocols in Human Genetics, Suppl. 60. (doi.org/10.1002/ 0471142905.hg0212s60) : le design des primers est réalisé par l'outil MassARRAY^{®} Assay Design version 4.0.0.2 permettant d'amplifier des fragments de moins de 120 bases et en sélectionnant des différences de masse entre allèles comprise entre 16 et 70 Da. Environ 15 ng d'ADN sont nécessaires pour génotyper les individus en utilisant la plateforme MassARRAY^{®} iPLEX (Agena Bioscience^{®}). Après une amplification en multiple, les produits de PCR sont analysés en spectrométrie de masse et la taille des allèles de chaque amplifiat est estimée à l'aide du logiciel de la suite MassARRAY^{®} System. Le génotypage porte sur les pools alléliques Q2demal, Q4demal, Q6demal et Q7demal qui contiennent uniquement des allèles liés au caractère démaliquant.

### Exemple 1 :

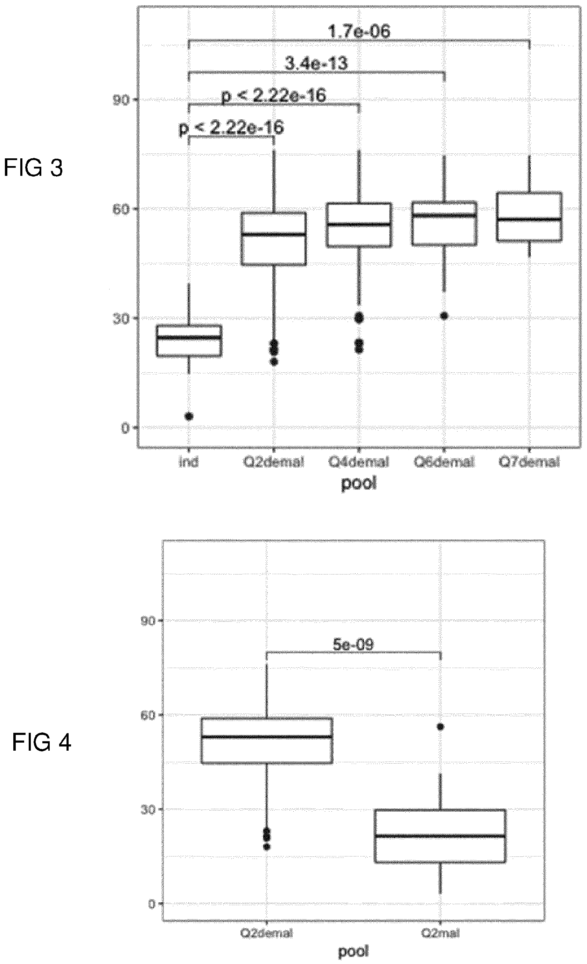

Dans le cas de la descendance de H3, les individus qui sont recherchés : les individus porteurs du pool allélique Q4demal qui comporte les allèles suivants : Q4demal : *YLL041C* (p.Lys158Glu) + *YGL008C* (p.Pro74Leu) + *YDL054C* (p.Ser63Leu) + *YDL231W* (p.Glu165Ala)*.* Parmi l'ensemble des descendants de H3 isolés, les individus présentant la combinaison allélique Q4demal sont analysés pour leurs propriétés fermentaires. Ces 53 individus présentent une valeur moyenne de 56.15% d'acide malique dégradé avec 25% des individus qui montrent une consommation d'acide malique supérieure à 61,72% d'acide malique dégradé ce qui est très supérieur à la valeur moyenne des populations H1 et H2 et à celle de levures commerciales (figure 3, sur laquelle la valeur en ordonnée correspond au pourcentage d'acide malique consommé calculé par rapport à la masse totale d'acide malique présent au départ, pour chacun des pools indiqués en abscisse).

Un des descendants de l'hybride H3, W647, est croisé avec un descendant de l'hybride H1 : X41. Le croisement entre W647 et X41 donne l'hybride H4. Ce nouveau croisement permet d'incorporer les allèles démaliquants des gènes *YKL029C, YOR090C* et *YBR218C* dans l'hybride H4. A partir de cet hybride des spores sont isolées par le même procédé faisant appel à l'hydrophobicité de leur paroi que le procédé décrit ci-avant. Dans le cas de cet hybride H4, plus de 1000 descendants sont isolés. Leur ADN est extrait et les individus sont génotypés par la méthode de *sequenom^{®}.*

### Exemple 2 :

Dans la descendance de l'hybride H4 on sélectionne les individus porteurs des pools alléliques Q2demal, et de préférence des pools alléliques Q4demal, et de manière d'avantage préférée des pools alléliques Q6demal, voire des pools alléliques Q7demal.

Parmi l'ensemble des descendants de H4 isolés, les individus qui présentent la combinaison allélique Q7demal : *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605) + *YOR090C* (p.Met419Leu) + *YBR218C* (p.Glu722Lys) sont analysés pour leur propriétés fermentaires. Ces 10 individus présentent une valeur moyenne de 58.59 % d'acide malique dégradé avec 25% des individus qui montrent une consommation d'acide malique supérieure à 64.42% d'acide malique dégradé (voir sur la figure 3), ce qui est très supérieur à la valeur moyenne des population H1 et H2 et à celle de levures commerciales (voir la figure 2).

### Synthèse de l'ensemble des résultats obtenus dans les conditions des exemples 1 et 2 :

Les résultats reportés sur la figure 3 (figure 3, sur laquelle la valeur en ordonnée correspond au pourcentage d'acide malique consommé calculé par rapport à la masse totale d'acide malique présent au départ, pour chacun des pools indiqués en abscisse) montrent que la sélection des individus de H3 et de H4 par les pools Q2demal, Q4demal, Q6demal et Q7demal permet d'obtenir des clones bien plus performants qu'un panel de 34 souches industrielles (pool indiqué tout à gauche du diagramme sur la figure 3) dont les valeurs d'acide malique dégradé en fin de fermentation sont comprises entre 5 et 35%. La valeur des levures industrielles a été mesurée dans les conditions de fermentation décrites dans l'article de Peltier et al., 2018, PLOS ONE, 13(1), 191353. (https://doi.org/10.1101/191353). Ces résultats sont confirmés par le test statistique de Wilcoxon, selon les conditions décrites dans Frank Wilcoxon, « Individual comparisons by ranking methods », Biometrics Bulletin (en), vol. 1, n° 6, 1945, p. 80-83, qui indique une différence très significative entre les levures industrielles et les clones H3 et H4.

Les résultats reportés sur la figure 4 (figure 4, sur laquelle la valeur en ordonnée correspond au pourcentage d'acide malique consommé calculé par rapport à la masse totale d'acide malique présent au départ, pour chacun des pools indiqués en abscisse) montrent que les individus de H3 et de H4 porteurs du pool Q2demal : *YLL041C* (p.Lys158Glu) + *YGL008C* (p.Pro74Leu), dégradent plus d'acide malique en fin de fermentation que ceux porteurs du pool Q2mal : *YLL041C* (p.Lys158) + *YGL008C* (p.Asp200Glu). Ces résultats sont confirmés par le test statistique de Wilcoxon qui indique une différence très significative entre les groupes.

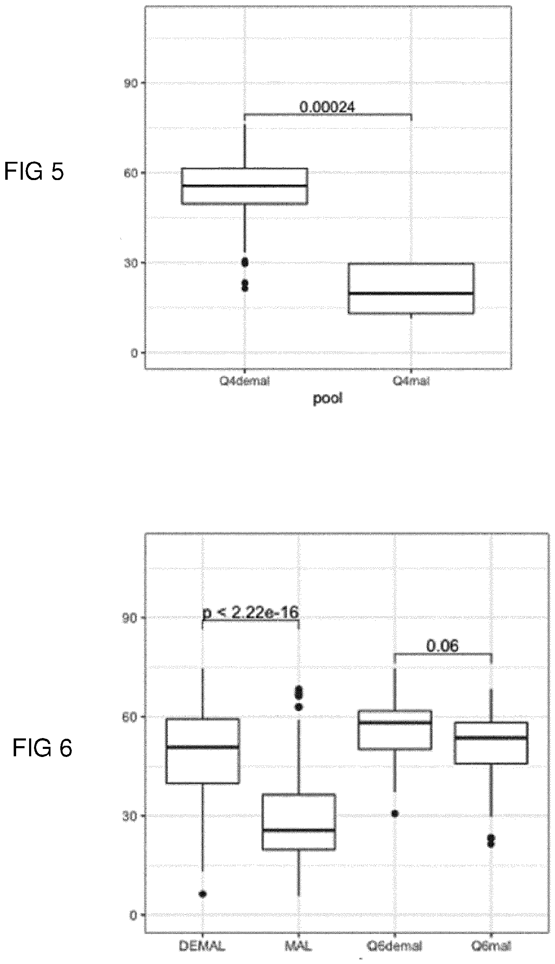

Les résultats reportés sur la figure 5 (figure 5, sur laquelle la valeur en ordonnée correspond au pourcentage d'acide malique consommé calculé par rapport à la masse totale d'acide malique présent au départ, pour chacun des pools indiqués en abscisse) montrent que les individus de H3 et de H4 porteurs du pool Q4demal : *YLL041C* (p.Lys158Glu) + *YGL008C* (p.Pro74Leu) + *YDL054C* (p.Ser63Leu) + *YDL237W* (p.Glu165Ala) dégradent plus d'acide malique en fin de fermentation que ceux porteurs du pool Q4mal : *YLL041C* (p.Lys158) + *YGL008C* (p.Asp200Glu) + *YDL054C* (p.Ser63) + *YDL237W* (p.Glu165). Ces résultats sont confirmés par le test statistique de Wilcoxon, selon les conditions décrites dans Frank Wilcoxon, « Individual comparisons by ranking methods », Biometrics Bulletin (en), vol. 1, n° 6, 1945, p. 80-83, qui indique une différence significative entre les groupes,

Les résultats reportés sur la figure 6 (figure 6, sur laquelle la valeur en ordonnée correspond au pourcentage d'acide malique consommé calculé par rapport à la masse totale d'acide malique présent au départ, pour chacun des pools indiqués en abscisse) montrent que, au sein de la descendance des hybrides H3 et H4, les allèles des gènes *YKL029C* et *YOR090C* ont un effet très significatif sur la dégradation de l'acide malique quelque soit les autres génotypes (visible dans les deux premières séries à gauche de la figure : le pool qualifié de DEMAL pour *YKL029C* (p.Ile605)+ *YOR090C* (p.Met419Leu), et le pool qualifié de MAL pour *YKL029C* (p.Ile605Val)+ *YOR090C* (p.Met419)). Ces résultats montrent l'intérêt d'incorporer les allèles *YKL029C* (p.Ile605Val) et *YOR090C* (p.Met419Leu) au pool Q4demal : ce pool correspond au pool Q6demal. Ainsi, les individus de H4 porteurs du pool Q6demal : *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605) + *YOR090C* (p.Met419Leu) dégradent plus d'acide malique en fin de fermentation que ceux porteurs du pool contrôle Q6c: *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605Val) + *YOR090C* (p.Met419). Ces résultats sont confirmés par le test statistique de Wilcoxon qui indique une différence significative entre les groupes au seuil de 10%.

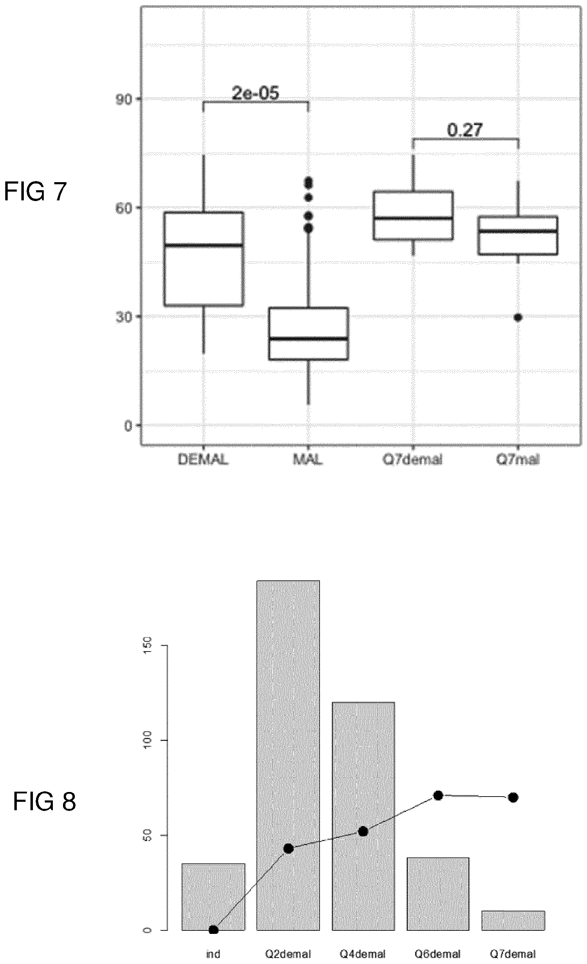

Les résultats reportés sur figure 7 montrent que, au sein de la descendance des hybrides H3 et H4, les allèles des gènes *YKL029C, YOR090C* et *YBR218C* ont un effet significatif sur la dégradation de l'acide malique quelque soit les autres génotypes (visible dans les deux premières séries à gauche de la figure : le pool qualifié de DEMAL pour *YKL029C* (p.Ile605)+ *YOR090C* (p.Met419Leu)+ *YBR218C* (p.Glu722Lys) et le pool qualifié de MAL pour *YKL029C* (p.Ile605Val)+ *YOR090C* (p.Met419)+ *YBR218C* (p.Glu722)). Ces résultats montrent l'intérêt d'incorporer les allèles *YKL029C* (p.Ile605Val) + *YOR090C* (p.Met419Leu) + YER218C(p.Glu722Lys) au pool Q4demal. Ce nouveau pool est nommé Q7demal. Ainsi, les individus de H4 porteurs du pool Q7demal : *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605Val) + *YOR090C* (p.Met419Leu) + *YBR218C* (p.Glu722Lys) dégradent plus d'acide malique en fin de fermentation que ceux porteur du pool contrôle Q7c: *YLL041C* (p.Lys158Glu) + *YGL008C* (p.Pro74Leu) + *YDL054C* (p.Ser63Leu) + *YDL237W* (p.Glu165Ala) + *YKL029C* (p.Ile605Val) + *YOR090C* (p.Met419) + *YBR218C* (p.Glu722).

Ainsi, la sélection sur la base du génotype des descendants des hybrides H3 et H4 à l'aide des pools Q2demal, Q4demal, Q6demal et Q7demal permet d'identifier des individus qui présentent une valeur d'acide malique dégradé très importante. Par contraste, la sélection d'individus parmi les mêmes populations présentant les pools Q2mal, Q4mal, Q6c et Q7c permet la sélection d'individus dégradant une masse d'acide malique moins importante calculée par rapport à la masse totale présente au départ (voir le tableau 4). Les effectifs et les valeurs moyennes des pools sont présentés dans le tableau 4.

**Tableau 4**

| Pool | % ac. Malique consommé | Variance | Nombre de souches descendants de H3 et H4 |
|---|---|---|---|
| Q2demal | 51.11123 | 141.95109 | 184 |
| Q2mal | 22.50 | 179 | 17 |
| Q4demal | 54.03593 | 113.09332 | 120 |
| Q4mal | 20.78336 | 76.49577 | 5 |
| Q6demal | 56.94121 | 88.02613 | 38 |
| Q6c | 50.23928 | 193.81062 | 23 |
| Q7demal | 58.59455 | 83.66503 | 10 |
| Q7c | 52.04401 | 120.84890 | 9 |

Les résultats obtenus et réportés sur la figure 8 montrent que la proportion d'individus qui consomment plus de 55% d'acide malique (valeur en ordonnée sur le diagramme de la figure 8) augmente selon que l'on utilise le pool d'allèle Q2demal, Q4demal Q6demal et Q7demal. Ce résultat est validé par un test de contingence au seil de 5% (test de khi2). Avec le pool Q7demal, la proportion de clones au-dessus de 55 est de 70% : ces résultats sont montrés sur la figure 8 ou les bâtons du diagramme correspondent au nombre d'individus testés, et la courbe le nombre d'individus identifiés comme étant au dessus dudit seuil de 55%.

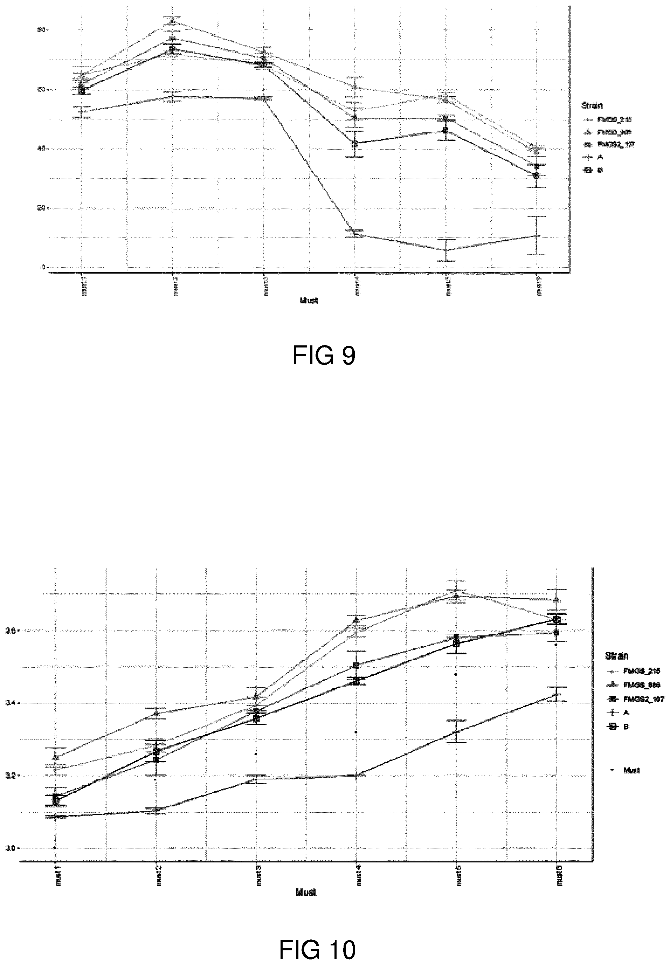

Grâce au procédé de croisement et de sélection mis en oeuvre tel que décrit ci-dessus, trois individus remarquables sont identifiés : FMGS_215, FMGS_889 et FMGS2_107. Les pourcentages de consommation d'acide malique de ces trois individus sont supérieurs à 65 % dans le vin issu du Merlot 2. On peut voir sur la figure 9 (les valeurs en ordonnées correspondant au pourcentage en acide malique) le comportement des souches sur trois moûts de Sauvignon blanc et trois moûts de Merlot (désignés sur la figure : « must 1 à 6 ») présentant des teneurs en d'acide malique initiales décroissantes : de 9.3 g/L à 2.3 g/L. Dans tous les cas les souches sélectionnées consomment plus d'acide malique que la souche B qui est la meilleure souche de notre collection pour ce caractère. L'acide L-malique est quantifié par dosage enzymatique après avoir observé l'arrêt complet de la fermentation alcoolique selon la méthode décrite dans Peltier, et al., 2018, PLOS ONE, 13(1), 191353 (https://doi.org/10.1101/191353) en utilisant un kit enzymatique conforme à la méthode OIV-OENO-599-2018 (Recueil des méthodes internationales d'analyses - Organisation internationale de la vigne et du vin). Ce dosage colorimétrique est basé sur la mesure de l'absorbance à 340 nm en spectrophotométrie UV.

Dans certaines conditions, le pourcentage d'acide malique consommé dépasse 80% (figure 9).

Par ailleurs, des expérimentations ont été réalisées en mesurant le pH des trois moûts de Sauvignon blanc et des trois moûts de Merlot (désignés sur les figures 9 et 10 : « must 1 à 6 ») qui montre que l'utilisation des trois souches FMGS_215, FMGS_889 et FMGS2_107 dans six matrices différentes permet de contrôler de façon remarquable le pH final des vins (voir la figure 10, où les valeurs en ordonnée correspondent au pH final qui est mesuré directement en utilisant un pH mètre préalablement étalonné). Entre les souches démaliquantes et la souche témoin A l'écart de pH peut atteindre 0.4 unités de potentiel Hydrogène.

**Sélection génétique d'une souche productrice d'acide malique dans** la **descendance de l'hybride H5:** un croisement entre les clones Z1 et X8 est ensuite effectué pour donner l'hybride H5 selon le même protocole que celui décrit ci-avant pour l'obtention des hybrides H1 et H2, H3 et H4. A partir de cet hybride H5, une préparation de spores est obtenue selon le protocole lié à l'hydrophobicité de leur paroi selon la méthode détaillée pour les descendances des hybrides H3 et H4. A partir de cette préparation, on isole 1000 descendants dont l'ADN est extrait selon des détails donnés pour la descendance des hybrides H3 et H4. Le génotype des descendants isolés des hybrides H3 H4 et H5 est évalué par la méthode du sequenom^{®} et porte sur les pools alléliques Q6mal et Q7mal qui contiennent uniquement des allèles liés au caractère maliquant. Les effectifs et les valeurs moyennes des pools sont présentés dans le tableau 5.

**Tableau 5**

| Pool | % ac. Malique consommé | Variance | Nombre de souches descendants de H3, H4 et H5 |
|---|---|---|---|
| Q6demal | 53,6 | 271,1 | 41 |
| Q6mal | -8,1 | 955,3 | 5 |
| Q7demal | 54,2 | 289,0 | 11 |
| Q7mal | -22,9 | 892,3 | 3 |

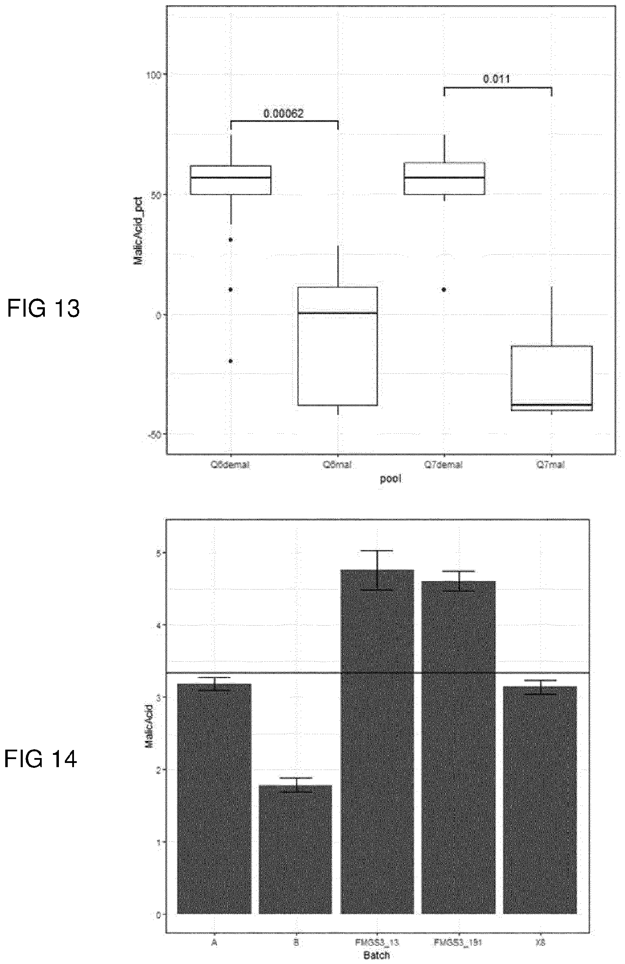

Parmi L'ensemble des descendants de H3, H4 et H5 isolés, les individus qui présentent la combinaison allélique Q6mal : *YLL041C* (p.Lys158) + *YGL008C*(p.Asp200Glu) + *YDL054C*(p.Ser63) + *YDL237W* (p.Glu165) + *YKL029C* (p.Ile605Val) + *YOR090C* (p.Met419) sont analysés pour leurs propriétés fermentaires. Certains individus porteurs du pool Q6mal présentent une production suplémentaire d'acide malique par rapport à la quantité initiale contenue dans le moût. Le paramètre est exprimé en pourcentage massique final calculé par rapport à la masse totale d'acide malique présent au départ. La valeur moyenne atteinte pour le pool Q6mal est de -8% c'est-à-dire que la levure produit 8 % de plus d'acide malique que celui initialement contenu dans le moût. Cette production est supérieure à celle de l'ensemble des levures commerciales dont la distribution est présentée figure 3. Les résultats synthétisés sur la figure 13 montrent la différence d'acide malique en fin de fermentation qui existe entre les souches porteuses des pools alléliques Q6mal et Q6demal chez l'ensemble des descendants. Ces résultats sont confirmés par le test statistique de Wilcoxon, selon les conditions décrites dans Frank Wilcoxon, « Individual comparisons by ranking methods », Biometrics Bulletin (en), vol. 1, n° 6, 1945, p. 80-83, qui indique une différence significative entre les groupes.

Parmi l'ensemble des descendants de H3, H4 et H5 isolés, les individus qui présentent la combinaison allélique Q7mal : *YLL041C* (p.Lys158) + *YGL008C* (p.Asp200Glu) + *YDL054C* (p.Ser63) + *YDL237W* (p.Glu165) + *YKL029C* (p.Ile605Val) + *YOR090C* (p.Met419) + *YBR218C* (p.Glu722) sont analysés pour leurs propriétés fermentaires. Les individus porteurs du pool Q7mal présentent une production supplémentaire d'acide malique par rapport à la quantité d'acide malique initiale contenue dans le moût. Ce paramètre est exprimé en pourcentage massique final calculé par rapport à la masse totale d'acide malique présent au départ. La valeur moyenne atteinte pour le pool Q7mal est de -22% c'est-à-dire que la levure produit 22% de plus d'acide malique que celui initialement contenu dans le moût. De façon tout a fait remarquable, les individus FMGS3_191 et FMGS3_13 produisent respectivement 37 et 42 % d'acide malique en plus que la teneur initialement contenue dans le moût. Sur la figure 13, on montre qu'il existe une différence entre les souches porteuses des pools alléliques Q7mal et Q7demal chez l'ensemble des descendants. Ces résultats sont confirmés par le test statistique de Wilcoxon, selon les conditions décrites dans Frank Wilcoxon, « Individual comparisons by ranking methods », Biometrics Bulletin (en), vol. 1, n° 6, 1945, p. 80-83, qui indique une différence significative entre les groupes.

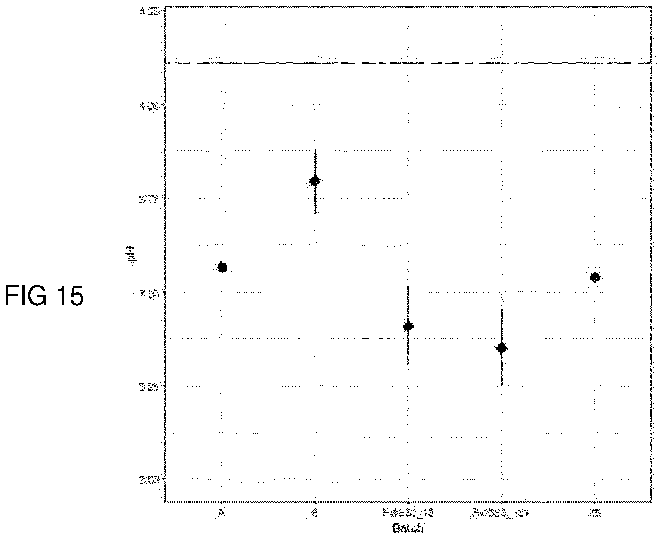

Dans l'exemple suivant, on vinifie un moût de cabernet sauvignon avec les souches FMGS3_191 et FMGS3_13 qui sont porteuses du pool Q7mal ainsi que les souches témoins A, B et X8. On mesure la teneur en acide malique en fin de la fermentation alcoolique. Sur la figure 14 les valeurs de l'axe des ordonnées sont exprimées en concentration massique d'acide malique par litre. Le trait horizontal indique la teneur initiale du moût en acide malique qui est de 3.34 g/L. Les souches FMGS3_191 et FMGS3_13 produisent une quantité remarquable d'acide malique très supérieure à celle de la souche X8 qui est le descendant le plus performant des hybrides H1 et H2. Ainsi ces deux souches produisent plus de 1.2 g/L voire plus de 1.4 g/L par rapport à la valeur initiale du moût. Cette production d'acide malique a un effet direct sur le pH des vins en fin de fermentation. Les résultats synthétisés sur la figure 15 montrent que les vins obtenus avec les souches manquantes sélectionnées ont des valeurs de pH inférieures de 0.2 unité de pH par rapport à la souche témoin A, voire de 0,4 unité de pH par rapport à la souche témoin B.

## Revendications

1. Utilisation dans la fermentation alcoolique du jus de raisin en vin d'une souche de levure de l'espèce *Saccharomyces cerevisiae* comprenant, par rapport à la souche de référence S288C, au moins la substitution d'acides aminés Asp200Glu dans la protéine PMA1 codée par le gène YGL008C.

2. Utilisation selon la revendication 1, pour modifier le pH du vin.

3. Utilisation selon la revendication 2, pour diminuer le pH du vin.

4. Utilisation selon l'une quelconque des revendications 1 à 3, pour augmenter la teneur en acide malique du vin en fin de la fermentation alcoolique.

5. Utilisation selon l'une quelconque des revendications 1 à 4, selon laquelle ladite souche de levure comprend en outre, par rapport à la souche de référence S288C, au moins la substitution d'acides aminés Ile605Val dans la protéine MAE1 codée par le gène YKL029C.

6. Utilisation selon l'une quelconque des revendications 1 à 5, selon laquelle ladite souche de levure comprend en outre, par rapport à la souche de référence S288C, au moins la substitution d'acides aminés Gly79Asp dans la protéine RDR1 codée par le gène YOR380W.

7. Utilisation selon l'une quelconque des revendications 1 à 6, selon laquelle ladite souche de levure comprend une lysine en position 158 de la protéine SDH2 codée par le gène YLL041C.

8. Utilisation selon l'une quelconque des revendications 1 à 7, selon laquelle ladite souche de levure comprend une sérine en position 63 de la protéine MCH1 codée par le gène YDL054C et un acide glutamique en position 165 de la protéine AIM6 codée par le gène YDL237W.

9. Utilisation selon l'une quelconque des revendications 1 à 8, selon laquelle ladite souche de levure comprend une méthionine en position 419 de la protéine PTC5 codée par le gène YOR090C.

10. Utilisation selon l'une quelconque des revendications 1 à 9, selon laquelle ladite souche de levure comprend un acide glutamique en position 722 de la protéine PYC2 codée par le gène YBR218C.

11. Souche de levure de l'espèce *Saccharomyces cerevisiae* comprenant, par rapport à la souche de référence S288C, au moins la substitution d'acides aminés Asp200Glu dans la protéine PMA1 codée par le gène YGL008C et la substitution d'acides aminés Ile605Val dans la protéine MAE1 codée par le gène YKL029C.

12. Souche de levure selon la revendication 11 comprenant en outre, par rapport à la souche de référence S288C, au moins la substitution d'acides aminés Gly79Asp dans la protéine RDR1 codée par le gène YOR380W.

13. Souche de levure selon la revendication 11 ou 12 comprenant une lysine en position 158 de la protéine SDH2 codée par le gène YLL041C.

14. Souche de levure selon l'une quelconque des revendications 11 à 13 comprenant une sérine en position 63 de la protéine MCH1 codée par le gène YDL054C et un acide glutamique en position 165 de la protéine AIM6 codée par le gène YDL237W.

15. Souche de levure selon l'une quelconque des revendications 11 à 14 comprenant une méthionine en position 419 de la protéine PTC5 codée par le gène YOR090C et/ou un acide glutamique en position 722 de la protéine PYC2 codée par le gène YBR218C.
